# EUROPEAN PATENT APPLICATION

(11) **EP 4 585 179 A1**
(43) Date of publication of application: **16.07.2025**
(21) Application number: 23863236.8
(22) Date of filing: 07.09.2023
(51) Int. Cl.: A61B 34/20, G06N 20/10

(54) **SURGERY ASSISTANCE PROGRAM, SURGERY ASSISTANCE DEVICE, AND SURGERY ASSISTANCE METHOD**

(30) Priority: 09.09.2022 JP 2022143972
(71) Applicant: Keio University, Tokyo, 108-8345 (JP)
(72) Inventor: TAKEUCHI, Masashi, Tokyo 160-8582 (JP)
(74) Representative: Epping - Hermann - Fischer
(86) International application number: PCT/JP2023/032611
(87) International publication number: WO 2024/053698

(57) **Abstract**

Notification of a risky action during surgery is accurately provided.

A program according to an embodiment of the present invention causes a computer to acquire a surgery image in which a surgical site is captured, detect a risky action from the acquired surgery image using a model generated by training with training data in which an input is a surgery image and an output is information related to a risky action, and notify that the risky action is detected, wherein the information related to the risky action includes region information of a structure altered due to the risky action in the surgery image.

## Description

### TECHNICAL FIELD

The present invention relates to a surgery assistance program, a surgery assistance apparatus, and a surgery assistance method.

### BACKGROUND ART

Conventionally, postoperative complications not only degrade the quality of life (QOL) of patients, but also lead to a deterioration in long-term prognosis and an increase in medical expenses, and thus, reduction of postoperative complications is one of the most important challenges for surgeons. It is known that the complication rate is strongly related to the experience of surgeons and the scale of facilities, and it is a well-known fact that surgical procedure during surgery is a cause of postoperative complications. Postoperative complications are often caused by insufficient recognition or insufficient attention to the situation during surgery rather than insufficient technical skill of the procedure.

As a method for reducing postoperative complications, for example, Patent Document 1 discloses a method of machine-learning surgery images and information on complication risks, and outputting risk analysis information by inputting surgery images at the time of inference.

### RELATED ART DOCUMENTS

### PATENT DOCUMENT

Patent Document 1: Japanese Unexamined Patent Publication No. 2021-029258

### SUMMARY OF THE INVENTION

### PROBLEMS TO BE SOLVED BY THE INVENTION

However, in Patent Document 1, the inference is made based on machine learning of surgery images and information on complication risks, and it is desired to be able to accurately notify when a risky action that may cause postoperative complications occurs during surgery.

Therefore, an object of the present invention is to accurately provide notification about a risky action during surgery.

### MEANS FOR SOLVING THE PROBLEMS

A program according to an embodiment of the present invention causes a computer to acquire a surgery image in which a surgical site is captured, detect a risky action from the acquired surgery image using a model generated by training with training data in which an input is a surgery image and an output is information related to a risky action, and provide notification that the risky action is detected, wherein the information related to the risky action includes region information of a structure altered due to the risky action in the surgery image.

According to the present invention, it is possible to accurately provide notification about a risky action during surgery.

### BRIEF DESCRIPTION OF THE DRAWINGS

[FIG. 1] FIG. 1 is a diagram showing an overall configuration of an embodiment of the present invention.
[FIG. 2] FIG. 2 is a functional block diagram of a surgery assistance apparatus according to an embodiment of the present invention.
[FIG. 3] FIG. 3 is a diagram for explaining region information of a structure altered due to a risky action in a surgery image according to an embodiment of the present invention.
[FIG. 4] FIG. 4 is a diagram for explaining region information of a structure altered due to a risky action in a surgery image according to an embodiment of the present invention.
[FIG. 5] FIG. 5 is a diagram for explaining region information of a structure altered due to a risky action in a surgery image according to an embodiment of the present invention.
[FIG. 6] FIG. 6 is a diagram for explaining region information of a structure altered due to a risky action in a surgery image according to an embodiment of the present invention.
[FIG. 7] FIG. 7 is a diagram for explaining region information of a structure altered due to a risky action in a surgery image according to an embodiment of the present invention.
[FIG. 8] FIG. 8 is a diagram for explaining region information of a structure altered due to a risky action in a surgery image according to an embodiment of the present invention.
[FIG. 9] FIG. 9 is a diagram for explaining region information of a structure altered due to a risky action in a surgery image according to an embodiment of the present invention.
[FIG. 10] FIG. 10 is a diagram for explaining machine learning according to an embodiment of the present invention.
[FIG. 11] FIG. 11 is a diagram for explaining machine learning according to an embodiment of the present invention.
[FIG. 12] FIG. 12 is a flowchart of a machine learning process according to an embodiment of the present invention.
[FIG. 13] FIG. 13 is a flowchart of a risky action notification process according to an embodiment of the present invention.
[FIG. 14] FIG. 14 is a flowchart of a machine training data creation process according to an embodiment of the present invention.
[FIG. 15] FIG. 15 is a flowchart of a risky action notification process using other information according to an embodiment of the present invention.
[FIG. 16] FIG. 16 is a flowchart of an evaluation process according to an embodiment of the present invention.
[FIG. 17] FIG. 17 is an example of information on which evaluation is based and evaluation results according to an embodiment of the present invention.
[FIG. 18] FIG. 18 is a hardware configuration diagram of a surgery assistance apparatus according to an embodiment of the present invention.

### DESCRIPTION OF EMBODIMENTS

Hereinafter, embodiments of the invention will be described with reference to the drawings.

### <Explanation of Terms>

- In the present specification, "surgery" may be any surgery, such as surgery performed only by a human, surgery performed by a human operating a medical device such as an endoscope (for example, laparoscopic surgery, thoracoscopic surgery, or the like), surgery performed by a human operating a robot, or surgery performed only by a robot. For example, surgery is a surgical operation that removes cancerous tissue, including primary lesions and lymph nodes, from tissue. For example, surgery is esophagectomy, gastrectomy, colectomy, hepatectomy, pancreatectomy, or endoscopic surgery (endoscopic submucosal dissection) for early cancer such as early gastrointestinal cancer.
- In the present specification, a "risky action" is an action that may cause postoperative complications among actions that occur during surgery. In other words, a "risky action" is an action (for example, an action of pulling the recurrent laryngeal nerve or an action of exposing a blood vessel) that may easily shift to an action that causes postoperative complications (for example, an action of paralyzing the recurrent laryngeal nerve or an action of bleeding from a blood vessel). The "risky action" can be said to be an action that is one step before an action that causes postoperative complications. For example, a risky action is an action of deforming a discretionarily chosen object (structure) constituting a human body, or an action of exposing a discretionarily chosen object (structure) constituting a human body (that is, causing the arbitrary object (structure) to appear in a surgery image (an image in which a surgical site is captured)). For example, a risky action is an action that causes recurrent nerve paralysis (i.e., an action of pulling the recurrent laryngeal nerve). In the present specification, the "structure" is a discretionarily chosen object constituting a human body. For example, a structure is an organ in the body (e.g., the recurrent laryngeal nerve, muscular layers, blood vessels), a body fluid (e.g., bile, intestinal fluid, blood that has bled), etc.

### <System Configuration>

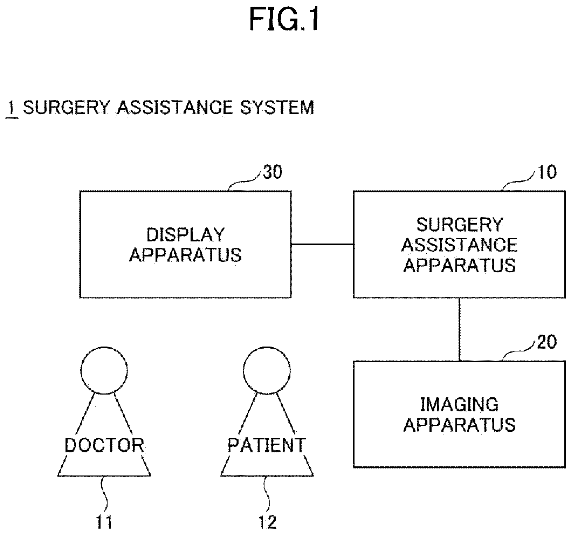

FIG. 1 is a diagram showing an overall configuration of an embodiment of the present invention. The surgery assistance system 1 includes a surgery assistance apparatus 10, an imaging apparatus 20, and a display apparatus 30. A case where the doctor 11 performs surgery on the patient 12 will be described. Each of these will be described below.

### <<Surgery Assistance Apparatus>>

The surgery assistance apparatus 10 is an apparatus that detects a risky action based on an image captured by the imaging apparatus 20 during surgery and notifies the doctor 11 that the risky action is detected (for example, causes the display apparatus 30 to display the risky action). The surgery assistance apparatus 10 includes one or a plurality of computers. The surgery assistance apparatus 10 can acquire an image captured by the imaging apparatus 20 from the imaging apparatus 20. The surgery assistance apparatus 10 can cause the display apparatus 30 to display information, such as a detection of a risky action.

### <<Imaging Apparatus>>

The imaging apparatus 20 is an apparatus that captures an image of a body part of the patient 12 undergoing a surgery (hereinafter, also referred to as a "surgical site"). Hereinafter, the image of the surgical site captured by the imaging apparatus 20 is also referred to as a "surgery image".

### <<Display Apparatus>>

The display apparatus 30 is a monitor, a display, or the like that displays information acquired from the surgery assistance apparatus 10. The doctor 11 can know that he/she is taking a risky action by viewing the information displayed on the display apparatus 30.

At least two of the surgery assistance apparatus 10, the imaging apparatus 20, and the display apparatus 30 may be implemented by one device.

### <Functional Block>

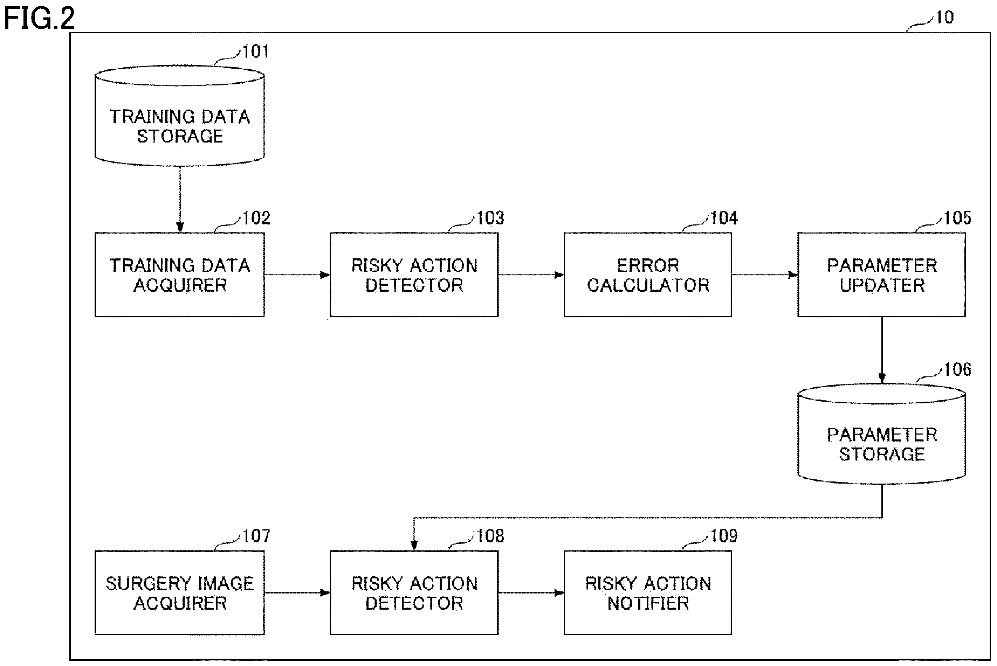

FIG. 2 is a functional block diagram of the surgery assistance apparatus 10 according to an embodiment of the present invention. The surgery assistance apparatus 10 includes a training data storage 101, a training data acquirer 102, a risky action detector 103, an error calculator 104, a parameter updater 105, a parameter storage 106, a surgery image acquirer 107, a risky action detector 108, and a risky action notifier 109. The surgery assistance apparatus 10 executes programs to function as the training data acquirer 102, the risky action detector 103, the error calculator 104, the parameter updater 105, the surgery image acquirer 107, the risky action detector 108, and the risky action notifier 109. Each of these will be described below.

The training data storage 101 stores training data. The training data is a surgery image in which a surgical site is captured and information related to a risky action for the surgery image.

The training data acquirer 102 acquires training data stored in the training data storage 101.

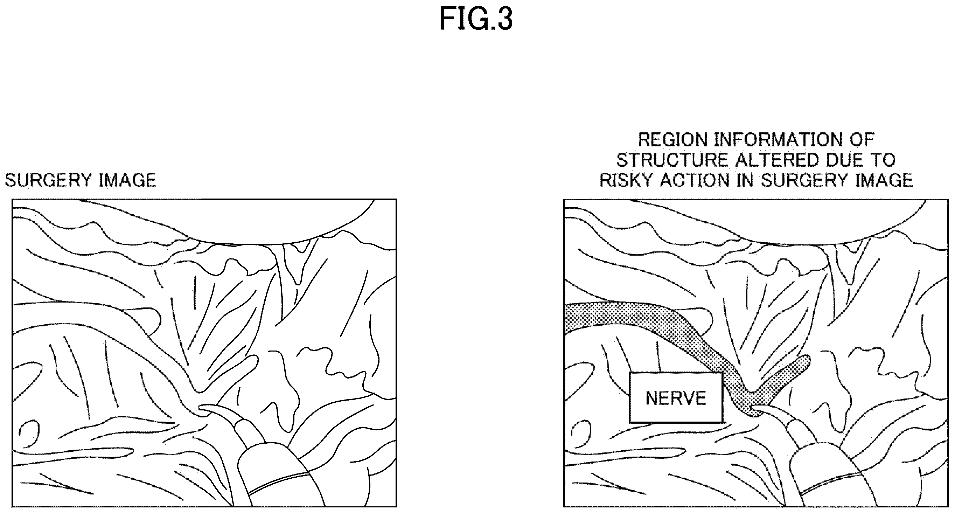

Herein, the "information related to a risky action" will be described. The "information related to a risky action" includes "region information of a structure altered due to a risky action in a surgery image (the region information is at least one of: segmentation information indicating a region of the structure in an image (information indicating a segmented structure); or frame information indicating a region of a structure in an image (information such as a rectangle surrounding a structure))" and "a label indicating whether a surgery image is an image indicating a risky action or an image indicating a non-risky action (an action that is not a risky action)". Hereinafter, the region information of a structure in a surgery image will be described in detail with reference to FIGS. 3 and 4 through 9.

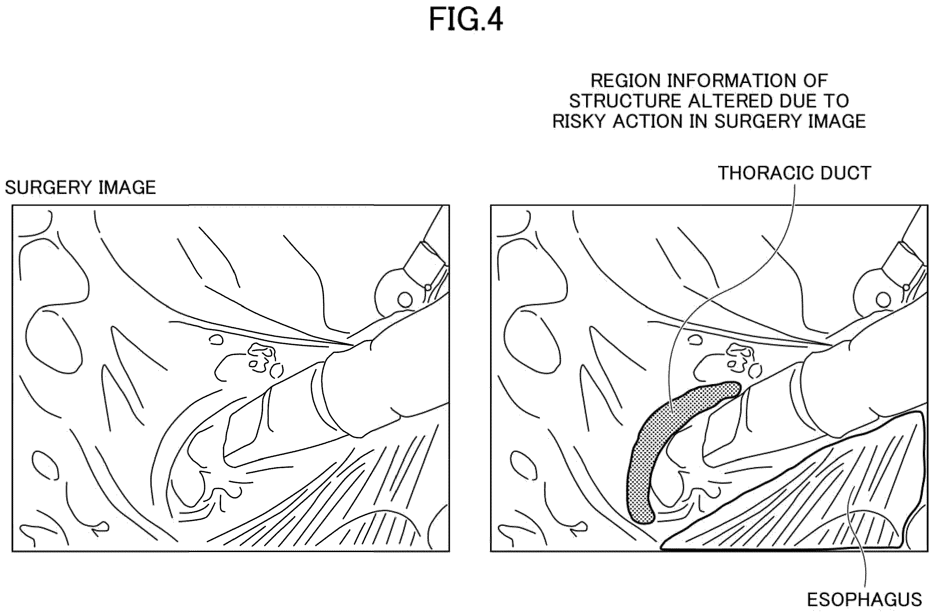

The region information of a structure altered due to a risky action in a surgery image is region information of a structure whose region in a surgery image changes as a result of the doctor 11 or the like taking a risky action (for example, a structure whose region changes in response to a change of the shape of the structure, or a structure whose region changes when the structure appears). Specifically, the region information of a structure altered due to a risky action in a surgery image includes at least one of: region information of a structure whose shape changes due to a risky action (i.e., region information of a structure that is deformed as a result of the doctor 11 or the like taking a risky action; this will be described with reference to FIG. 3); or region information of a structure that appears in a surgery image due to a risky action (i.e., region information of a structure that appears in a surgery image as a result of the doctor 11 or the like taking a risky action; this will be described with reference to FIGS. 4 through 9).

FIG. 3 is a diagram for explaining region information of a structure altered due to a risky action in a surgery image according to an embodiment of the present invention. FIG. 3 illustrates a state in which a nerve is deformed as a result of a doctor 11 or the like taking a risky action.

The "surgery image" in FIG. 3 is an image of a surgical site captured during surgery.

In the "region information of a structure altered due to a risky action in a surgery image" of FIG. 3, region information of a structure altered due to a risky action in a surgery image (that is, a structure clearly shown in a surgery image) is added to the surgery image. Such region information of a structure altered due to a risky action is used for training a machine learning model.

FIG. 4 is a diagram for explaining region information of a structure altered due to a risky action in a surgery image according to an embodiment of the present invention. FIG. 4 illustrates a state in which the thoracic duct appears as a result of the doctor 11 or the like taking a risky action.

The "surgery image" in FIG. 4 is an image of a surgical site captured during surgery.

In the "region information of a structure altered due to a risky action in a surgery image" of FIG. 4, region information of the thoracic duct (a structure that appears in a surgery image due to a risky action) is added to the surgery image. In an esophagectomy, region information of the thoracic duct (a structure that appears in a surgery image due to a risky action) can be used for training a machine learning model.

In this way, in an esophagectomy, when the thoracic duct, which is normally difficult to recognize and easily damaged (a chylothorax is caused when damaged), is recognized in the image during a lymph node dissection, the operator is notified that the operator might be taking a risky action, and it is thereby possible to prevent serious complications.

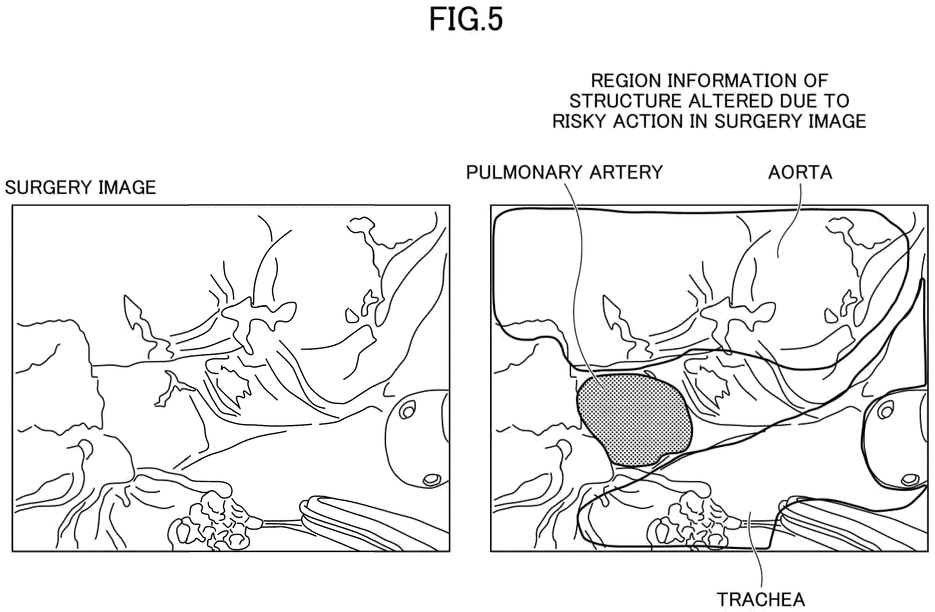

FIG. 5 is a diagram for explaining region information of a structure altered due to a risky action in a surgery image according to an embodiment of the present invention. FIG. 5 illustrates a state in which the pulmonary artery appears as a result of the doctor 11 or the like taking a risky action.

The "surgery image" in FIG. 5 is an image of a surgical site captured during surgery.

In the "region information of a structure altered due to a risky action in a surgery image" of FIG. **5****,** region information of the pulmonary artery (a structure that appears in a surgery image due to a risky action) is added to the surgery image. In an esophagectomy, region information of the pulmonary artery (a structure that appears in a surgery image due to a risky action) can be used for training a machine learning model.

In this way, in an esophagectomy, when the pulmonary artery, which is normally difficult to recognize and is easily damaged (if damaged, there is a risk of massive bleeding and death), is recognized in the image during a lymph node dissection, the operator is notified that the operator might be taking a risky action, and it is thereby possible to prevent serious complications.

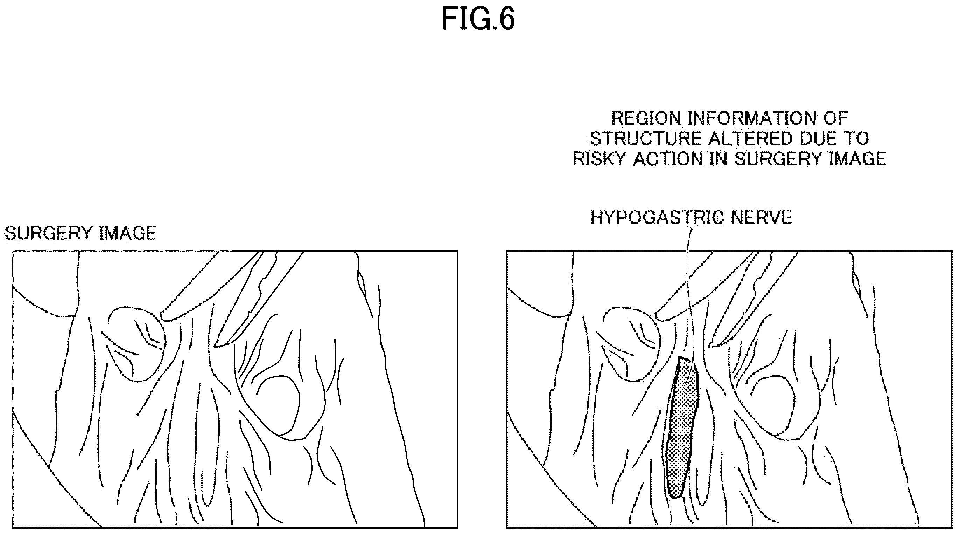

FIG. 6 is a diagram for explaining region information of a structure altered due to a risky action in a surgery image according to an embodiment of the present invention. FIG. 6 illustrates a state in which the hypogastric nerves appear as a result of the doctor 11 or the like taking a risky action.

The "surgery image" in FIG. 6 is an image of a surgical site captured during surgery.

In the "region information of a structure altered due to a risky action in a surgery image" of FIG. 6, region information of the hypogastric nerves (a structure that appears in a surgery image due to a risky action) is added to the surgery image. In a colectomy, region information of the pulmonary artery (a structure that appears in a surgery image due to a risky action) can be used for training a machine learning model.

In this way, in a colectomy, when a nerve that should not be exposed (if damaged, an ejaculatory disorder, erectile disorder, or voiding disorder is caused) is recognized in the image during a lymph node dissection, the operator is notified that the operator might be taking a risky action, and it is thereby possible to prevent serious complications.

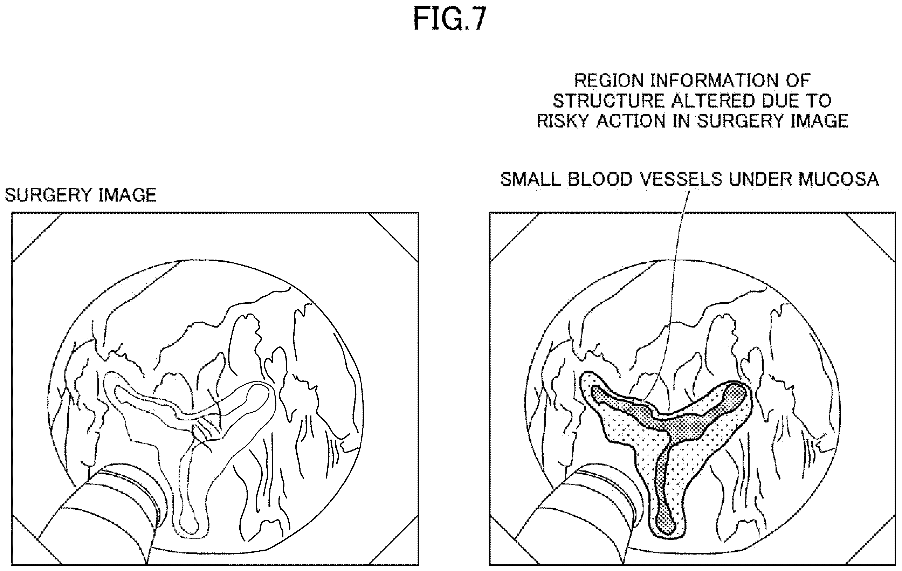

FIG. 7 is a diagram for explaining region information of a structure altered due to a risky action in a surgery image according to an embodiment of the present invention. FIG. 7 illustrates a state in which small blood vessels appear under a mucosa as a result of the doctor 11 or the like taking a risky action.

The "surgery image" in FIG. 7 is an image of a surgical site captured during surgery.

In the "region information of a structure altered due to a risky action in a surgery image" of FIG. 7, region information of small blood vessels (a structure that appears in a surgery image due to a risky action) is added to the surgery image. In an endoscopic surgery for early gastrointestinal cancer, region information of small blood vessels (a structure that appears in a surgery image due to a risky action) can be used for training a machine learning model.

In this way, in an endoscopic surgery for early gastrointestinal cancer, when small blood vessels, which are usually easily overlooked and easily damaged (bleeding is caused when damaged), are recognized in the image, the operator is notified that the operator might be taking a risky action, and it is thereby possible to prevent serious complications.

The "region information of a structure in the surgery image which changes due to a risky action" may be region information of a structure covered with a mucous membrane or the like (region information of a mucous membrane or the like may also be used as in FIG. 7, or region information of a mucous membrane or the like need not to be used), or may be region information of a structure not covered with a mucous membrane or the like.

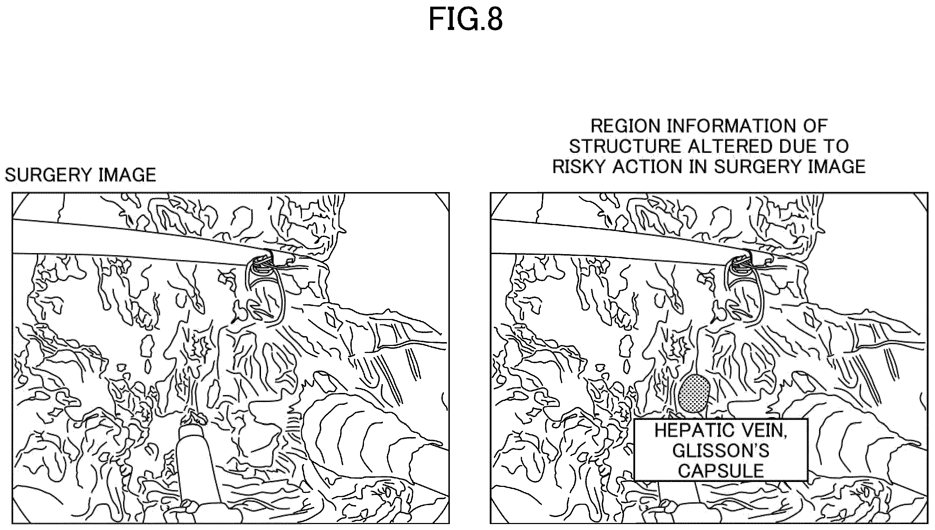

FIG. 8 is a diagram for explaining region information of a structure altered due to a risky action in a surgery image according to an embodiment of the present invention. FIG. 8 illustrates a state in which a hepatic vein and a Glisson's capsule appear as a result of the doctor 11 or the like taking a risky action.

The "surgery image" in FIG. 8 is an image of a surgical site captured during surgery.

In the "region information of a structure altered due to a risky action in a surgery image" of FIG. 8, region information of a hepatic vein and a Glisson's capsule (a structure that appears in a surgery image due to a risky action) is added to the surgery image. In a hepatectomy, region information of a hepatic vein and a Glisson's capsule (a structure that appears in a surgery image due to a risky action) can be used for training a machine learning model.

In this way, in a hepatectomy, when a hepatic vein and a Glisson's capsule, which are usually difficult to recognize and easily damaged (biliary fistula and hemorrhage are caused when damaged), are recognized in the image, the operator is notified that the operator might be taking a risky action, and it is thereby possible to prevent serious complications.

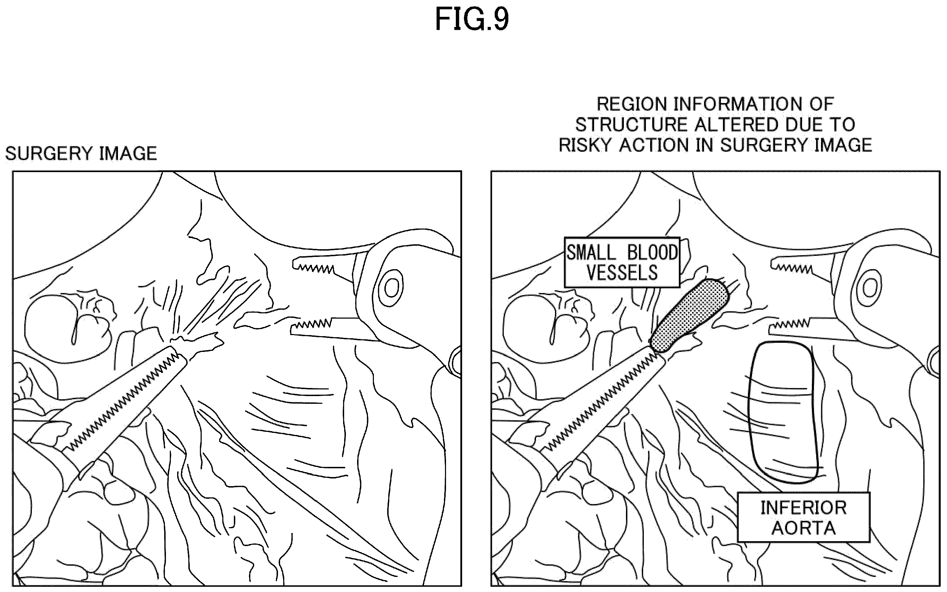

FIG. 9 is a diagram for explaining region information of a structure altered due to a risky action in a surgery image according to an embodiment of the present invention. FIG. 9 illustrates a state in which small blood vessels appear as a result of the doctor 11 taking a risky action.

The "surgery image" in FIG. 9 is an image of a surgical site captured during surgery.

In the "region information of a structure altered due to a risky action in a surgery image" of FIG. 9, region information of small blood vessels (a structure that appears in a surgery image due to a risky action) is added to the surgery image. In a pancreatectomy, region information of small blood vessels (a structure that appears in a surgery image due to a risky action) can be used for training a machine learning model.

In this way, in a pancreatectomy, when a branch of a large blood vessel such as the inferior vena cava, which is usually overlooked and which is easily damaged (when damaged, a large hemorrhage is caused), is recognized in the image, the operator is notified that the operator might be taking a risky action, and it is thereby possible to prevent serious complications.

The region information of a structure may be generated by a human designating the region of the structure in the surgery image, or through inference using a machine learning model trained with surgery images in which regions of structures are designated by a human. In addition, a human may determine that the action is risky action or non-risky action by viewing the surgery image and may attach a label, or as will be described later, a label may be attached using information detected by a sensor.

The description returns to FIG. 2. The risky action detector 103 detects a risky action using the model being trained.

### (Example 1)

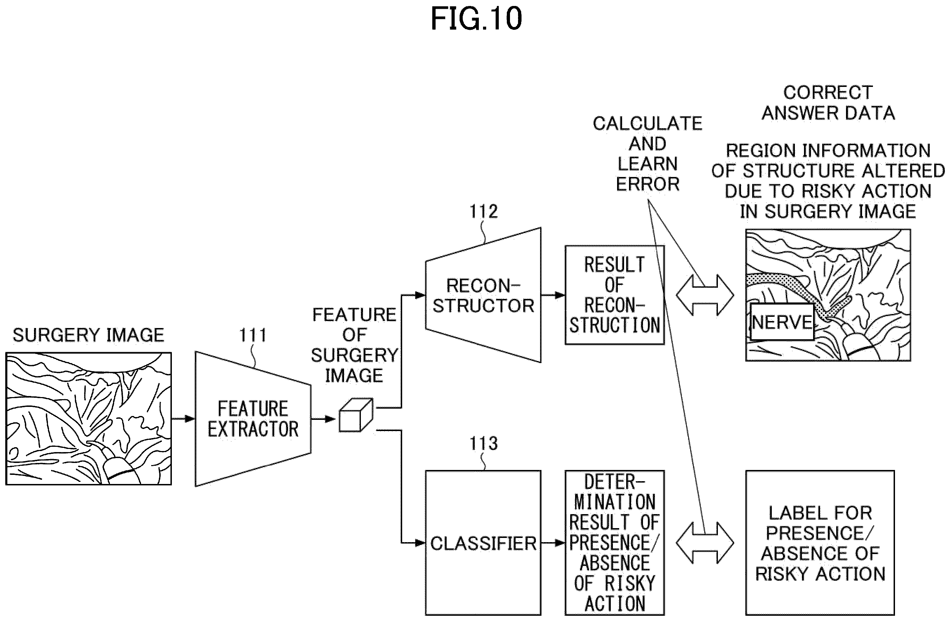

In Example 1 described with reference to FIG. 10, the risky action detector 103 inputs a surgery image acquired by the training data acquirer 102 to the model being trained (the feature extractor 111 and the reconstructor 112 in FIG. 10), and outputs region information of a structure in the surgery image. The risky action detector 103 inputs a surgery image acquired by the training data acquirer 102 to the model being trained (the feature extractor 111 and the classifier 113 in FIG. 10), and outputs the presence or absence of a risky action (whether or not an action is or is not a risky action). The presence or absence of a risky action may be expressed by a ratio.

### (Example 2)

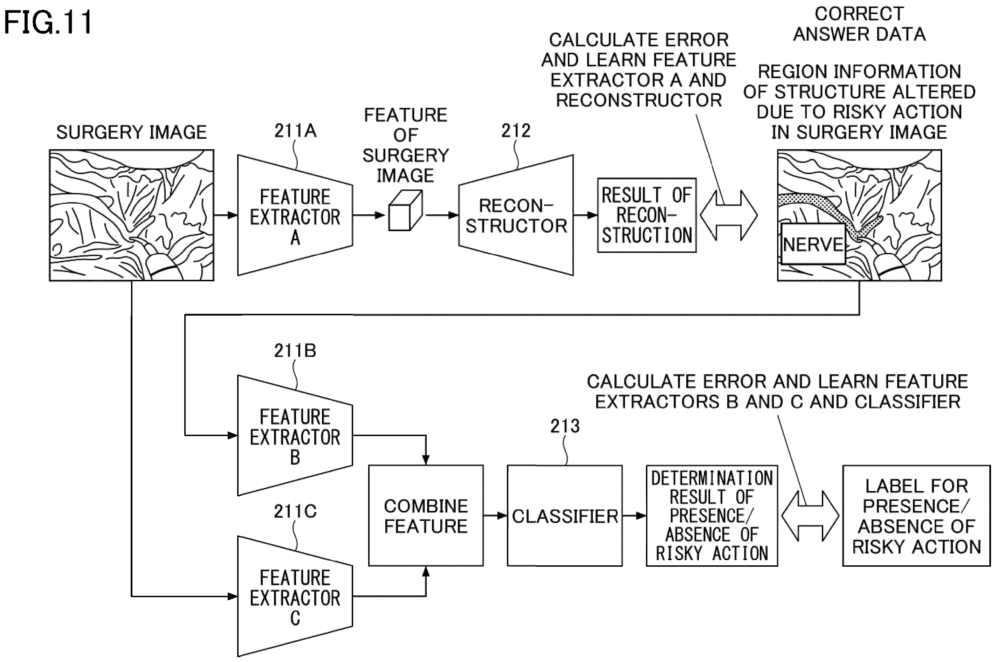

In Example 2 described with reference to FIG. 11, the risky action detector 103 inputs a surgery image acquired by the training data acquirer 102 to the model being trained (the feature extractor A 211A and the reconstructor 212 in FIG. 11), and outputs region information of a structure in the surgery image. The risky action detector 103 inputs a surgery image acquired by the training data acquirer 102 and region information of a structure in a surgery image reconstructed by the reconstructor 212 to the model being trained (the feature extractor B 211B, the feature extractor C 211C, and the classifier 213 in FIG. 11), and outputs the presence or absence of a risky action (whether an action is or is not a risky action). The presence or absence of a risky action may be expressed by a ratio.

The error calculator 104 calculates an error between the information related to a risky action detected from the surgery image by the risky action detector 103 and the information related to a risky action of the surgery image acquired by the training data acquirer 102.

The parameter updater 105 updates parameters of the model being trained so that the error calculated by the error calculator 104 becomes small, and stores the updated parameters in the parameter storage 106.

The parameter storage 106 stores the parameters of the model updated by the parameter updater 105.

The machine learning process will be described in detail below with reference to FIG. 10 (Example 1) and FIG. 11 (Example 2).

FIG. 10 is a diagram for explaining the machine learning according to an embodiment of the present invention (Example 1). The feature extractor 111 extracts a feature from a surgery image which is training data, the reconstructor 112 reconstructs region information of a structure or a surgery image including region information of a structure from the feature, and the classifier 113 determines whether an action is a risky action or not from the feature. In other words, in the inference in Example 1, the classifier 113 classifies the surgery image using the feature extracted by the feature extractor 111 at the time of machine learning. Each of these will be described below.

The feature extractor (also referred to as an "encoder") 111 extracts a feature of a surgery image from the surgery image. Specifically, the feature extractor 111 extracts a feature by reducing the dimension of a surgery image.

The reconstructor (also referred to as a "decoder") 112 reconstructs region information of a structure or a surgery image including region information of a structure based on the feature of the surgery image extracted by the feature extractor 111 (the region information of a structure to be reconstructed may only be region information of a structure altered due to a risky action, or may include not only region information of a structure altered due to a risky action but also region information of other structures). An error between the result of the reconstruction by the reconstructor 112 and the region information of a structure of the training data (correct answer data) (i.e., region information of a structure altered due to a risky action in a surgery image) is calculated, and learning is performed (i.e., the parameters are updated) so that the error becomes small.

The classifier 113 determines whether the surgery image is an image indicating that an action is a risky action or an image indicating that an action is not a risky action based on the feature of the surgery image extracted by the feature extractor 111. An error between the result of the determination by the classifier 113 and the label of the training data (correct answer data) (i.e., a label indicating an action is a risky action or the label indicating that an action is not a risky action) is calculated, and learning is performed (i.e., the parameters are updated) so that the error becomes small.

FIG. 11 is a diagram for explaining the machine learning according to an embodiment of the present invention (Example 2). The feature extractor A 211A extracts a feature from a surgery image as the training data, and the reconstructor 212 reconstructs region information of a structure or a surgery image including region information of a structure from the feature. The feature extractor B 211B extracts a feature from the region information of a structure or the surgery image including region information of a structure reconstructed by the reconstructor 212. The feature extractor C 211C extracts a feature from a surgery image as the training data. The classifier 213 determines whether an action is a risky action or not from the feature obtained by combining the feature extracted by the feature extractor B 211B and the feature extracted by the feature extractor C 211C. In other words, in Example 2, at the time of inference, the feature extractor A 211A and the reconstructor 212 reconstruct region information of a structure from a surgery image, and the classifier 213 classifies the surgery image using a feature of the surgery image and a feature of the reconstructed region information of a structure. Each of these will be described below.

The feature extractor (also referred to as an "encoder") A 211A extracts a feature of a surgery image from the surgery image. Specifically, the feature extractor A 211A extracts a feature by reducing the dimension of a surgery image.

The reconstructor (also referred to as a "decoder") 212 reconstructs region information of a structure or a surgery image including region information of a structure based on the feature of the surgery image extracted by the feature extractor A 211A (the region information of a structure to be reconstructed may only be region information of a structure altered due to a risky action, or may include not only region information of a structure altered due to a risky action but also region information of other structures). An error between the result of the reconstruction by the reconstructor 212 and the region information of a structure of the training data (correct answer data) (i.e., region information of a structure altered due to a risky action in a surgery image) is calculated, and learning is performed (i.e., the parameters are updated) so that the error becomes small.

The feature extractor (also referred to as an "encoder") B 211B extracts a feature of region information of a structure from region information of a structure reconstructed by the reconstructor 212. Specifically, the feature extractor B 211B extracts a feature by reducing the dimension of region information of a structure reconstructed by the reconstructor 212.

The feature extractor (also referred to as an "encoder") C 211C extracts a feature of a surgery image from the surgery image. Specifically, the feature extractor C 211C extracts a feature by reducing the dimension of a surgery image.

The classifier 213 determines whether the surgery image is an image indicating that an action is a risky action or an image indicating that an action is not a risky action based on the feature obtained by combining the feature extracted by the feature extractor B 211B and the feature extracted by the feature extractor C 211C. An error between the result of the determination by the classifier 213 and the label of the training data (correct answer data) (i.e., a label indicating an action is a risky action or the label indicating that an action is not a risky action) is calculated, and learning is performed (i.e., the parameters are updated) so that the error becomes small.

By training the model in this way, the surgery assistance apparatus 10 can reflect the degree of recognition of a structure altered due to a risky action during surgery and a positional relationship of such a structure to the detection of a risky action. In other words, in the present invention, since a risky action is detected using the result of segmentation of an anatomical structure and the frame information **(i.e.,** the region information of each structure) as "attention", the information of each structure can contribute to the determination of whether or not an action is a risky action.

For example, the region information of a structure altered due to a risky action in a surgery image is information of the shape of the recurrent laryngeal nerve in an esophagectomy (i.e., region information of the recurrent laryngeal nerve). In this case, when the recurrent laryngeal nerve is deformed by some action, the operator is notified that the operator might be taking a risky action, and it is thereby possible to prevent serious complications.

For example, the region information of a structure altered due to a risky action in a surgery image is information of at least one of a muscle layer or a blood vessel in an endoscopic submucosal dissection (i.e., region information of at least one of a muscle layer or a blood vessel). In this case, when a muscular layer or a blood vessel that should not be exposed is recognized, the operator is notified that the operator might be taking a risky action, and it is thereby possible to prevent serious complications.

For example, the region information of a structure altered due to a risky action in a surgery image is information of at least one of bile, intestinal fluid, or bleeding (bled blood) in an esophagectomy (i.e., region information of at least one of bile, intestinal fluid, or bleeding). In this case, when bile, intestinal fluid, or bleeding is recognized in a certain amount or more during surgery, the operator is notified that the operator might be taking a risky action, and it is thereby possible to prevent serious complications.

The accuracy of the model inference was 46.8% in the case of machine learning without using region information of a structure, and the accuracy of the model inference was 88.0% in the case of machine learning using region information of a structure. In this way, by using region information of a structure, the accuracy of the model inference can be improved.

The description returns to FIG. 2. The surgery image acquirer 107 acquires a surgery image (for example, a moving image) captured by the imaging apparatus 20 during surgery.

The risky action detector 108 detects a risky action based on the surgery image acquired by the surgery image acquirer 107. Specifically, the risky action detector 108 inputs the surgery image acquired by the surgery image acquirer 107 to a machine learning model (specifically, a model using the parameters stored in the parameter storage 106), and outputs the presence or absence of a risky action (whether an action is or is not a risky action). The presence or absence of a risky action may be represented by a ratio (the probability of a risky action or the probability of not a risky action may be output using a classification model, or the probability of a risky action or the probability of not a risky action may be output using a regression model).

The risky action notifier 109 provides notification about the risky action detected by the risky action detector 108 in real time (for example, causes the display apparatus 30 to display a notification). For example, the risky action notifier 109 causes the detection of a risky action to be displayed on the surgery image acquired by the surgery image acquirer 107. For example, the risky action notifier 109 displays "Risky action is being taken" or the like.

### <Method of the Process>

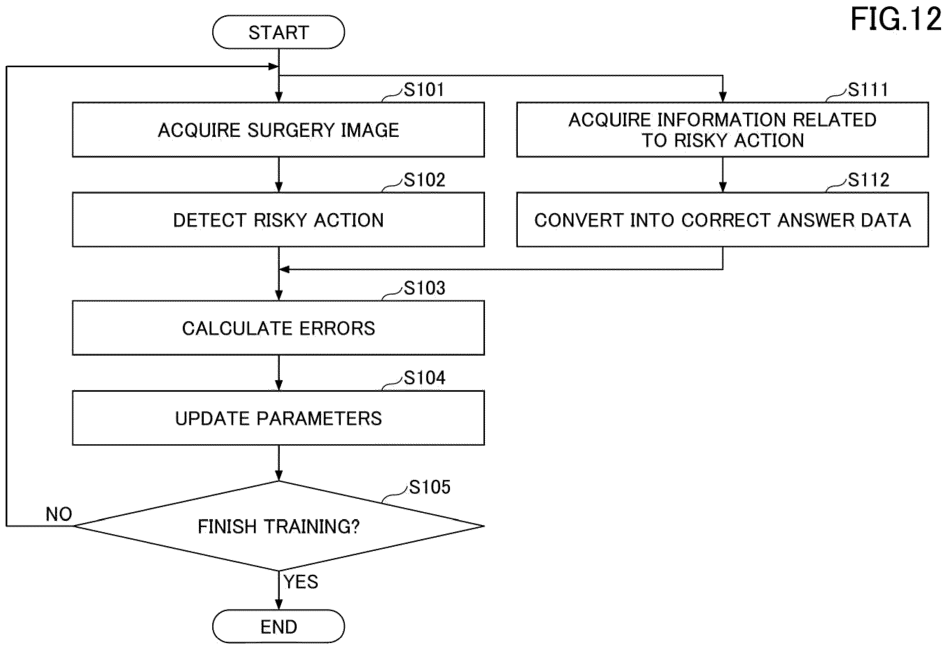

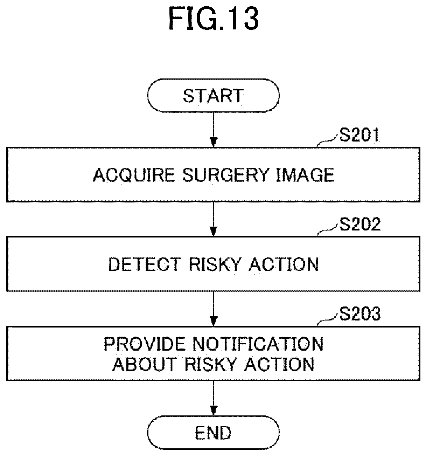

Hereinafter, a method of the learning process will be described with reference to FIG. 12, and a method of the risky action notification process will be described with reference to FIG. 13.

FIG. 12 is a flowchart of a machine learning process according to an embodiment of the present invention.

In step 101 (S101), the surgery assistance apparatus 10 acquires a surgery image, which is training data.

In step 102 (S102), the surgery assistance apparatus 10 detects a risky action using a machine learning model being trained.

In Example 1 described with reference to FIG. 10, the surgery assistance apparatus 10 inputs the surgery image acquired in S101 to the model being trained (the feature extractor 111 and the reconstructor 112 in FIG. 10), and outputs region information of a structure in the surgery image. The surgery assistance apparatus 10 inputs the surgery image acquired in step S101 to the model being trained (the feature extractor 111 and the classifier 113 in FIG. 10), and outputs the presence or absence of a risky action (whether an action is or is not a risky action).

In Example 2 described with reference to FIG. 11, the surgery assistance apparatus 10 inputs the surgery image acquired in S101 to the model being trained (the feature extractor A 211A and the reconstructor 212 in FIG. 11), and outputs region information of a structure. The surgery assistance apparatus 10 inputs the surgery image acquired in S101 and the region information of the structure reconstructed by the reconstructor 212 to the model being trained (the feature extractor B 211B, the feature extractor C 211C, and the classifier 213 in FIG. 11), and outputs the presence or absence of a risky action (whether an action is or is not a risky action) .

In step 111 (S111), the surgery assistance apparatus 10 acquires information related to a risky action of the surgery image as training data (i.e., the surgery image acquired in S101) (specifically, region information of a structure altered due to a risky action in a surgery image, and a label indicating whether a surgery image is an image indicating a risky action or an image indicating a non-risky action (an action that is not a risky action)).

In step 112 (S112), the surgery assistance apparatus 10 converts the data acquired in S111 into correct answer data. Specifically, the surgery assistance apparatus 10 arranges the data acquired in S111 in the format of an output of the model.

In step 103 (S103), the surgery assistance apparatus 10 calculates an error between information related to the risky action detected in S102 and the correct answer date in S112.

In step 104 (S104), the surgery assistance apparatus 10 updates the parameters of the model being trained so that the error calculated in S103 becomes small, and stores the updated parameters in the parameter storage 106.

In step 105 (S105), the surgery assistance apparatus 10 determines whether or not to end the training. If the training is not to be ended, the process returns to S101 and S111 to acquire a next set of training data.

FIG. 13 is a flowchart of the risky action notification process according to an embodiment of the present invention.

In step 201 (S201), the surgery assistance apparatus 10 acquires a surgery image (for example, a moving image) captured by the imaging apparatus 20 during surgery.

In step 202 (S202), the surgery assistance apparatus 10 detects a risky action on the basis of the surgery image acquired in S201. Specifically, the surgery assistance apparatus 10 inputs the surgery image acquired in S201 to the model generated as illustrated in FIG. 12, and outputs the presence or absence of a risky action (whether an action is or is not a risky action). If a risky action is detected, the process proceeds to step 203.

In the first embodiment described with reference to FIG. 10, the classifier 113 classifies the surgery image using the feature extracted by the feature extractor 111 at the time of machine learning.

In other words, in Example 2 described with reference to FIG. 11, at the time of inference, the feature extractor A 211A and the reconstructor 212 reconstruct region information of a structure from a surgery image, and the classifier 213 classifies the surgery image using a feature of the surgery image and a feature of the reconstructed region information of a structure.

In step 203 (S203), the surgery assistance apparatus 10 provides notification in real time that the risky action is detected in S202 (for example, causes the display apparatus 30 to display a notification). For example, the surgery assistance apparatus 10 causes the detection of the risky action to be displayed on the surgery image acquired in S201.

### <Effects>

In one embodiment of the present invention, during a surgical procedure, when a risky action that may cause postoperative complications occurs, notification of the risky action is provided on a surgery image in real time, and thus the operator can immediately stop the risky action and, as a result, postoperative complications can be avoided.

Other embodiments will be described below. A plurality of embodiments described below may be implemented in combination.

### (Creation of Training data)

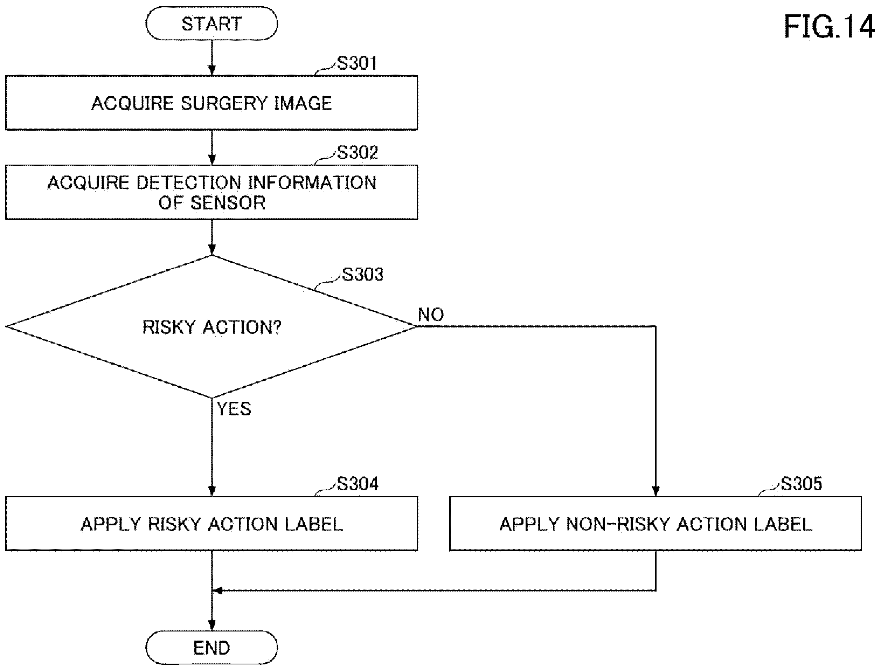

The surgery assistance apparatus 10 can create training data using information detected by a sensor. Specifically, the surgery assistance apparatus 10 can attach a label to a surgery image, which is training data, based on information detected by a sensor (for example, a sensor of the intraoperative neuromonitoring system). For example, information detected by a sensor is information regarding a risky action regarding whether or not an action that causes recurrent nerve paralysis, such as an action of excessively pulling the recurrent laryngeal nerve and surrounding tissues, an action of damaging the recurrent laryngeal nerve, or the like, has occurred. Labeling of training data will be described with reference to FIG. 14.

FIG. 14 is a flowchart of a machine training data creation process according to an embodiment of the present invention.

In step 301 (S301), the surgery assistance apparatus 10 acquires a surgery image (for example, a moving image) captured by the imaging apparatus 20 during surgery.

In step 302 (S302), the surgery assistance apparatus 10 acquires information detected by a sensor during surgery in which the surgery image of S301 is acquired (for example, information detected by a sensor of the intraoperative neuromonitoring system).

In step 303 (S303), the surgery assistance apparatus 10 determines whether or not the information acquired in S302 is a notification of a risky action. In the case where the information acquired in S302 is a notification of a risky action, the process proceeds to step 304, and in the case where the information acquired in S302 is not a notification of a risky action, the process proceeds to step 305.

In step 304 (S304), the surgery assistance apparatus 10 attaches a label indicating a risky action to a surgery image corresponding to a time range in which a notification of a risky action is made in S303.

In step 305 (S305), the surgery assistance apparatus 10 attaches a label indicating that the action is not a risky action to a surgery image corresponding to a time range in which a notification that an action is not a risky action is made in S303.

In this way, by creating training data using information detected by a sensor, it is possible to eliminate the burden of creating training data by a human attaching labels indicating the presence or absence of a risky action to surgery images.

### (Information of Structure Not Clearly Shown in Surgery Image)

The information related to a risky action may further include information of a structure not clearly shown in a surgery image. In order to detect a risky action, it is important to ascertain information on a structure that does not appear in a surgery image. In this regard, information on a structure that does not appear in a surgery image is acquired and used for determining whether an action is a risky action or not. For example, information of a structure not clearly shown in a surgery image is information indicating a region in a surgery image in which the recurrent laryngeal nerve is predicted to run.

In this case, in addition to the "region information of a structure altered due to a risky action in a surgery image" described with reference to FIGS. 10 and 11, "information of a structure not clearly shown in a surgery image (for example, a region in a surgery image where the recurrent laryngeal nerve is predicted to run) (the region information is at least one of segmentation information indicating a region of a structure in an image (information indicating a segmented structure) or frame information indicating a region of a structure in an image (information of a rectangle, etc. surrounding a structure)" is used for training.

In this way, by using information of a structure not clearly shown in a surgery image, it is possible to prevent unnecessary dissection operation, reduce complications, reduce the amount of bleeding, and shorten the surgical time.

### (Detection and Notification of Risky Action Using Other Information)

In an embodiment of the present invention, other information may also be used to detect and provide notification of a risky action. Hereinafter, a case where information of a preoperative patient's state is used will be described.

### ((Information of Pre-Operative Patient's State))

The surgery assistance apparatus 10 can detect a risky action by further using information of a preoperative patient's state. Whether or not an action is a risky action in a surgery depends on a patient's state. Therefore, information of a patient's state is acquired before a surgery and used for determining whether or not an action is a risky action. For example, the information on a preoperative patient's state is a preoperative CT image or a patient background when a risky action that may cause a complication of pneumonia is detected in an esophagectomy and notification of the risky action is provided.

Thus, by using information on a preoperative patient's state, it is possible to select a surgical procedure according to the state and risk of the patient.

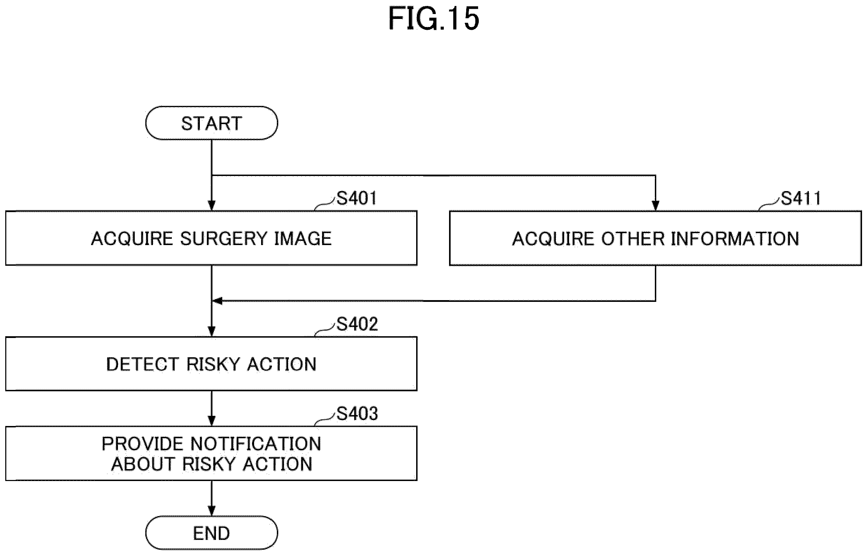

FIG. 15 is a flowchart of the risky action notification process using other information according to an embodiment of the present invention.

In step 401 (S401), the surgery assistance apparatus 10 acquires a surgery image captured by imaging apparatus 20 during surgery.

In step 411 (S411), the surgery assistance apparatus 10 acquires other information (for example, information of a preoperative patient's state).

In step 402 (S402), the surgery assistance apparatus 10 detects a risky action on the basis of the surgery image acquired in S401 and the other information acquired in S411. Specifically, when the model is trained, other information (for example, information of a preoperative patient's state) is input in addition to a surgery image. The surgery assistance apparatus 10 inputs the surgery image acquired in S401 and the other information acquired in S411 to the model generated by machine learning with training data in which an input is a surgery image and other information (e.g., information of a preoperative patient's state) and an output is information related to a risky action, and outputs the presence or absence of a risky action (whether an action is or is not a risky action). If a risky action is detected, the process proceeds to step 403.

In step 403 (S403), the surgery assistance apparatus 10 provides notification in real time that the risky action is detected in S402 (for example, causing the display apparatus 30 to display a notification).

### (Evaluation of Procedure)

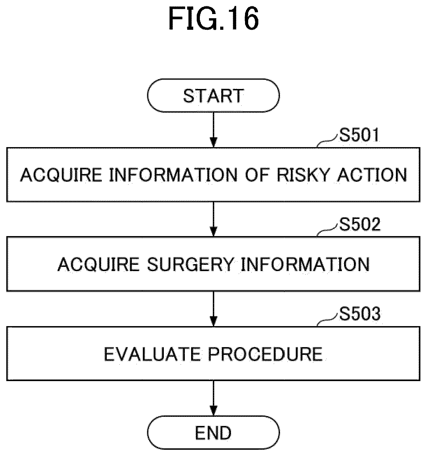

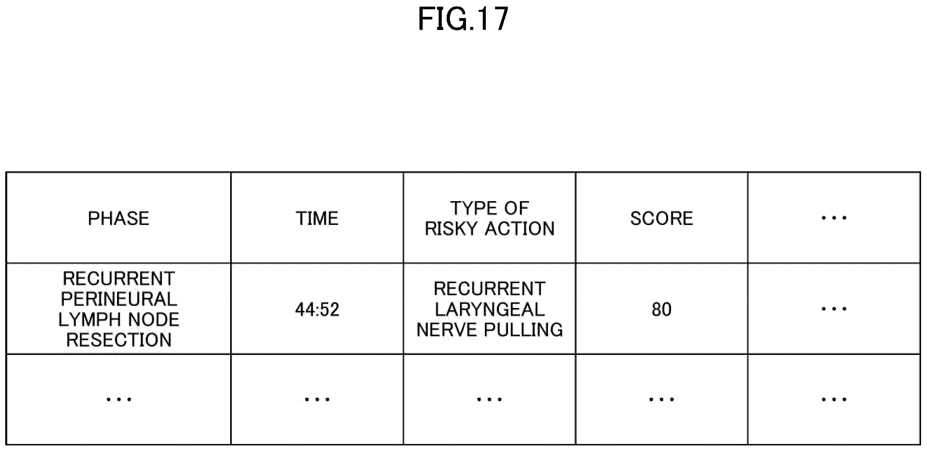

The surgery assistance apparatus 10 can evaluate the procedure of the operator, such as a surgeon or an endoscopist, based on the information related to a risky action that is output by inputting a surgery image to the model. Hereinafter, the evaluation of the procedure will be described with reference to FIGS. 16 and 17.

FIG. 16 is a flowchart of an evaluation process according to an embodiment of the present invention.

In step 501 (S501), the surgery assistance apparatus 10 acquires information related to the risky action that is output by inputting a surgery image to the model.

In step 502 (S502), the surgery assistance apparatus 10 acquires information related to a surgery. The information related to a surgery may be an entire surgery time, a surgery time in each phase, data obtained by recognizing the movement of the instrument or the operator, or the like.

In step 503 (S503), the surgery assistance apparatus 10 evaluates the procedure performed by the operator based on the information acquired in S501 and S502. For example, the surgery assistance apparatus 10 may evaluate the procedure of the operator, using machine learning.

FIG. 17 is an example of information on which evaluation is based and evaluation results according to an embodiment of the present invention. For example, the information on which the evaluation is based is the time of each phase of the surgery (for example, recurrent perineural lymphadenectomy), the type of risky action (for example, recurrent nerve traction), and the like. For example, the evaluation result may be a score.

Through evaluating the procedure, it is possible to perform feedback of the procedure of the surgery after the surgery and to use the procedure for surgery education.

### <Hardware Configuration>

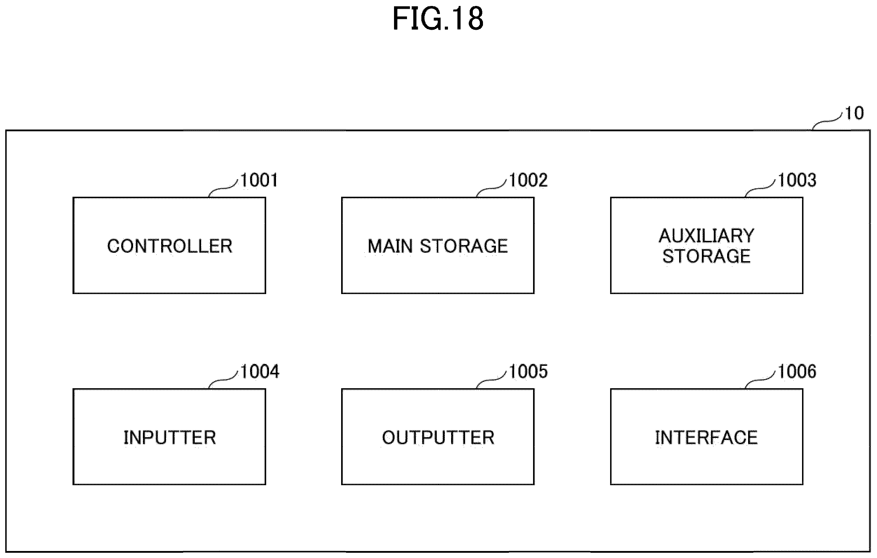

FIG. 18 is a hardware configuration diagram of the surgery assistance apparatus 10 according to an embodiment of the present invention. The surgery assistance apparatus 10 can include a controller 1001, a main storage 1002, an auxiliary storage 1003, an inputter 1004, an outputter 1005, and an interface 1006. Each of these will be described below.

The controller 1001 is a processor (for example, a central processing unit (CPU), a graphics processing unit (GPU), or the like) that executes various programs installed in the auxiliary storage 1003.

The main storage 1002 includes a nonvolatile memory (read only memory (ROM)) and a volatile memory (random access memory (RAM)). The ROM stores various programs, data, and the like necessary for the controller 1001 to execute various programs installed in the auxiliary storage 1003. The RAM provides a work area in which various programs installed in the auxiliary storage 1003 are loaded when the controller 1001 executes the programs.

The auxiliary storage 1003 is an auxiliary storage device that stores various programs and information used when the various programs are executed.

The inputter 1004 is an input device through which an operator of the surgery assistance apparatus 10 inputs various instructions to the surgery assistance apparatus 10.

The outputter 1005 is an output device that outputs an internal state of the surgery assistance apparatus 10 and the like.

The interface 1006 is a communication device for connecting to a network and communicating with other devices.

Although the embodiments of the present invention have been described in detail, the present invention is not limited to the above-described specific embodiments, and various modifications and changes can be made within the scope of the gist of the present invention described in the claims.

This international application claims priority based on Japanese Patent Application No. 2022-143972 filed on September 9, 2022, and the entire contents of Japanese Patent Application No. 2022-143972 are incorporated herein by reference.

### REFERENCE SIGNS LIST

- 1: Surgery assistance system
- 10: Surgery assistance apparatus
- 20: Imaging apparatus
- 30: Display apparatus
- 11: Doctor
- 12: Patient
- 101: Training data storage
- 102: Training data acquirer
- 103: Risky action detector
- 104: Error calculator
- 105: Parameter updater
- 106: Parameter storage
- 107: Surgery image acquirer
- 108: Risky action detector
- 109: Risky action notifier
- 111: Feature extractor
- 112: Reconstructor
- 113: Classifier
- 211A: Feature extractor A
- 211B: Feature extractor B
- 211C: Feature extractor C
- 212: Reconstructor
- 213: Classifier
- 1001: Controller
- 1002: Main storage
- 1003: Auxiliary storage
- 1004: Inputter
- 1005: Outputter
- 1006: Interface

## Claims

1. A program that causes a computer to:
acquire a surgery image in which a surgical site is captured;
detect a risky action from the acquired surgery image by using a machine learning model generated by training with training data in which an input is a surgery image and an output is information related to a risky action; and
notify that the risky action is detected, wherein
the information related to the risky action includes region information of a structure that is altered due to the risky action in the surgery image.

2. The program according to claim **1,** wherein
the region information of a structure altered due to the risky action in the surgery image includes at least one of
region information of a structure whose shape changes due to the risky action, or
region information of a structure that appears in the surgery image due to the risky action.

3. The program according to claim 1 or **2,** causing the computer to:
cause the detection of the risky action to be displayed on the acquired surgery image.

4. The program according to claim 1 or **2,** wherein
the training data is created using information detected by a sensor.

5. The program according to claim 1 or **2,** wherein
the information related to the risky action includes information of a structure not clearly shown in the surgery image.

6. The program according to claim 1 or **2,** causing the computer to:
detect the risky action further using information of a preoperative patient's state.

7. The program according to claim 1 or **2,** causing the computer to:
evaluate a procedure of a surgery of an operator based on the information related to the risky action that is output by inputting the acquired surgery image to the model.

8. The program according to claim 1 or **2,** wherein
the risky action is an action that causes recurrent nerve paralysis.

9. A surgery assistance apparatus, comprising:
a surgery image acquirer configured to acquire a surgery image in which a surgery site is captured;
a risky action detector configured to detect a risky action from the acquired surgery image by using a machine learning model generated by training with training data in which an input is a surgery image and an output is information related to a risky action; and
a risky action notifier configured to notify that the risky action is detected, wherein
the information related to the risky action includes region information of a structure altered due to the risky action in the surgery image.

10. A method performed by a surgery assistance apparatus, the method comprising:
a step of acquiring a surgery image in which a surgical site is captured;
a step of detecting a risky action from the acquired surgery image by using a machine learning model generated by training with training data in which an input is a surgery image and an output is information related to a risky action; and
a step of notifying that the risky action is tested; wherein
the information related to the risky action includes region information of a structure altered due to the risky action in the surgery image.
